# EUROPEAN PATENT SPECIFICATION

(11) **EP 1 552 785 B1**
(45) Date of publication and mention of the grant of the patent: **08.08.2012**
(21) Application number: 05250034.5
(22) Date of filing: 07.01.2005
(51) Int. Cl.: A61B 5/04

(54) **Body surface bio-potential sensor having multiple electrodes and apparatus comprising the body surface bio-potential sensor**
Mehrelektroden-Senor zur Erfassung von Biosignalen an Körperoberfläche und Gerät mit einem solchen Sensor
Capteur à électrodes multiples pour mesurer un signal biologique de la surface corporelle et dispositif comprenant le capteur

(30) Priority: 08.01.2004 KR 2004001097
(43) Date of publication of application: 13.07.2005
(73) Proprietor: Samsung Electronics Co., Ltd., Suwon-si, Gyeonggi-do (KR)
(72) Inventor: Jeong-hwan Lee, Yeongtong-gu, Suwon-si Gyeonggi-do (KR); Kun-soo, Shin, Seongnam-si, Gyeonggi-do (KR); Jin-sang Whang, Jangan-gu, Suwon-si, Gyeonggi-do (KR); Kyung-ho Kim, Yongin-si, Gyeonggi-do (KR); Hyung-sok Yeo, Giheung-eub gyeoggi-do (KR)
(74) Representative: Greene, Simon Kenneth

(56) References cited:
- EP-A- 1 060 704
- EP-A- 1 488 740
- US-A- 4 899 753
- US-A- 4 969 468
- US-A1- 2002 028 991

## Description

The present invention relates to a body surface bio-potential sensor having multiple electrodes and an apparatus having the body surface bio-potential sensor.

Conventionally, biomedical signal sensors use a pad provided with one or a plurality of electrodes, and the electrodes are connected to a signal processing unit through separate lead wires. Each electrode has a snap connected to a terminal of the lead wires. Such a connection configuration may generate abnormal noise caused by friction between the snap and the terminal of the lead wires when a person being examined moves. The abnormal noise may reduce the accuracy of detecting the biomedical signals. Furthermore, the lead wires connecting the signal processing unit to the electrodes may limit the movement of the person, and, especially, during an emergency, hinder a first-aid treatment.

On the other hand, a gel material is spread on a conventional electrode for promoting a smooth electrical contact considering a high resistance characteristic of the human skin. However, the gel material causes an uncomfortable sensation and, in some cases, may cause an eruption on the human skin.

US Patent No. 6,201,981, "*Electrode for Measuring Biomedical Signal and Electrode Support Device for Measuring a Biomedical Signal*," discloses methods of connecting an electrode to lead wires and of reducing a noise level. However, since a detection unit is also connected through the lead wires according to this method, movement of an examiner as well as an examined person are inevitably limited.

A method of using a transmitter, disclosed in U.S. Patent No. 6,267,723, has tried to solve such inconveniences caused by the lead wires. However, a gel material should be also used to contact an electrode to the human skin.

US 4,969,468 describes an electrode array for making electrical connection with tissue, especially nerves.

According to an aspect of the present invention, there is provided a body surface bio-potential sensor according to claim 1.

According to another aspect of the present invention, there is provided an apparatus for detecting biomedical signals according to claim 3.

A terminal for outputting electrical signals to an external unit is provided on the other surface of the membrane and electrically connected to the electrodes.

A complementary terminal may be provided on one surface of the transmitter, and the transmitter may receive electrical signals through the terminal from a multiple electrode patch.

The membrane and the transmitter have magnetic means for combining the membrane and the transmitter by a mutual attraction force.

The present invention thus provides a multiple electrode patch for detecting biomedical signals and a biomedical signal detection system having the same, by which biomedical signals can be stably detected from an examined person without using a gel material.

The above and other features and advantages of the present invention will become more apparent by describing in detail exemplary embodiments thereof with reference to the attached drawings in which:
FIG. 1 is a top view showing a sensor according to an exemplary embodiment of the present invention;
FIG. 2 is a rear view of the sensor of FIG. 1;
FIG. 3 is a cross-sectional view taken along a line A-A' in FIG. 2; and
FIG. 4 is a partially enlarged cross-sectional view of the sensor of FIG. 1.

The present invention will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those skilled in the art. Like reference numerals in the drawings denote like elements, and thus their description will not be repeated.

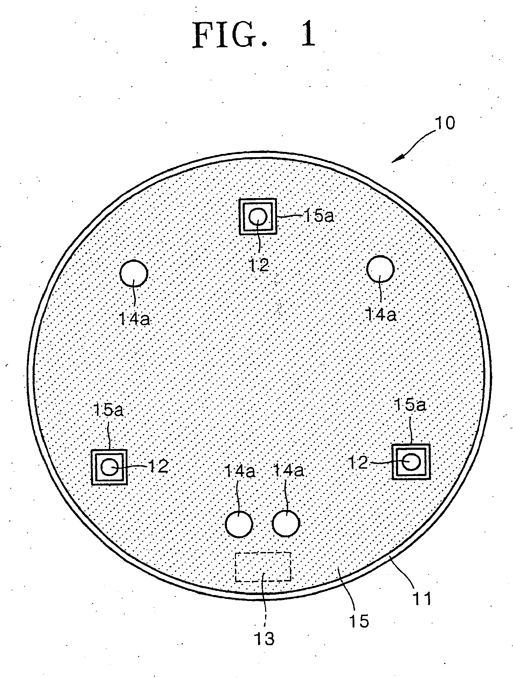

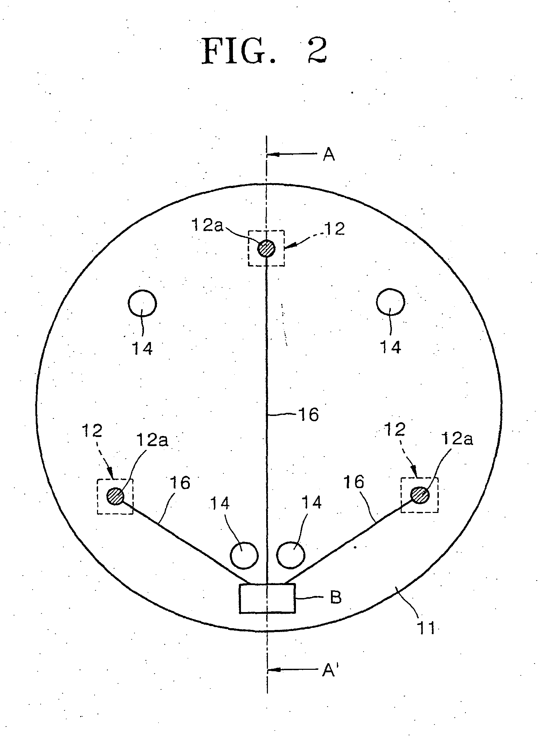

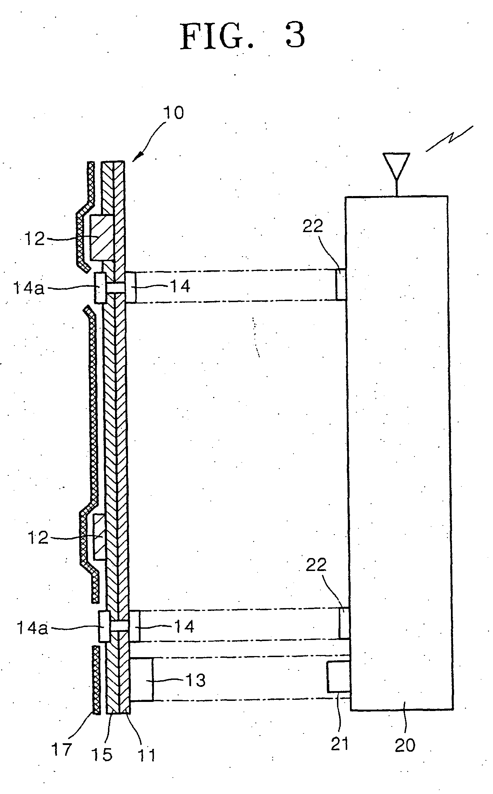

FIG. 1 is a top view showing a sensor according to the present invention, and FIG. 2 is a rear view thereof. In addition, FIG. 3 is a cross-sectional view taken along a line A-A' in FIG. 1.

A sensor 10 uses a disk-shaped membrane 11, which is a flexible printed circuit board (PCB) having a wire layer 16 formed thereon, as a substrate. The membrane 11 has a plurality of electrodes 12 attached to one side thereof. In this embodiment, three electrodes 12 are arranged in a triangular shape, and are connected to a terminal 13 provided close to edges of the membrane 11 through the wire layer 16 formed on a front or rear surface of the membrane 11. The terminal 13 is a connector complementarily paired with other terminal 21 provided in a transmitter 20. The front surface of the membrane 11 is provided with a cohesive sheet 15, which is to be attached to the human skin. The cohesive sheet 15 is provided with thru-holes 15a in positions corresponding to the electrode 12 and a magnet 14.

Typically, the magnet 14 has a button shape and is provided on a back side of the membrane 11 to be fixed with respect to the membrane 11, and a fixing element 14a for fixing the magnet 14 is provided on an opposite side of the membrane 14. In addition, the transmitter 20 is also provided with a button type permanent magnet 22 corresponding to the magnet 14 provided in the sensor 10.

According to such a configuration, the sensor 10 and the transmitter 20 can be combined with each other by using the permanent magnets 14 and 11. In this case, the two terminals 13 and 21 are interconnected with each other, thereby allowing transmission of electrical signals and supplementing a bonding force between the sensor 10 and the transmitter 20. Reference numeral 17 in FIG. 3 is a release base film for protecting the cohesive sheet 15.

When the sensor 10 is attached to a patch 5, the electrodes 12 directly contact to the human skin without applying a gel material thereto, thereby increasing a signal detection sensitivity. Moreover, the sensor 10 and the transmitter 20 do not require a separate lead wire for connecting them. Therefore, it is possible to reduce mechanical-electrical noise caused by the lead wire.

Also, it is possible to directly connect a plurality of wires to a part of a human body in order to detect low intensity electrical signals with high efficiency and transmit the signals to units such as an electrocardiograph. Furthermore, an examiner and an examined person can move freely without being hindered by the lead wires.

On the other hand, a direct unstable contact between the biomedical electrodes and the human skin may generate complicated voltages or impedances, thereby restricting an accurate measurement of the electrical signals. To prevent this problem, conventionally, a conductive hydro-gel adhesive has been used to attach the electrodes to the human skin via a conductive polymer medium in order to obtain stable electrical signals through close adhesion as well as not to harm the human skin. However, such an adhesive solidifies after a predetermined time, so that its function is significantly degraded. To solve this problem, a plurality of needles are provided on the electrodes to improve an electrical contact stability. The needles are formed on the surfaces of the electrodes by using a micro electro-mechanic system (MEMS) technology.

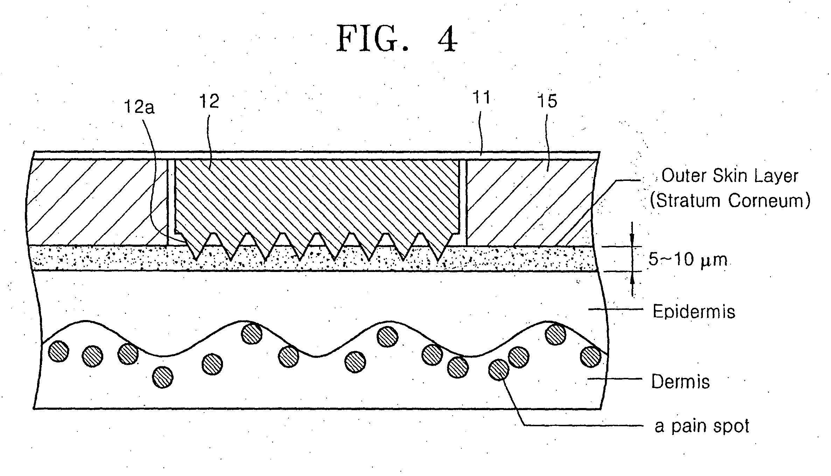

FIG. 4 is a partially enlarged cross-sectional view of the sensor of FIG. 1, which shows the arrangement of the electrodes 12 with respect to the skin. A plurality of needles 12a are provided on the surfaces of the electrodes 12. The needle 12a has a height of, for example, 5 micrometers or lower, enough to pass through the stratum corneum of the skin but not to reach the epidermis. This is because the needle 12a should not stimulate the epidermis having pain spots. In other words, the needle 12a of the electrode 12 is penetrated into the skin with a predetermined depth while the examined person does not feel the pain as well as electrical contact sensitivity is increased. Therefore, the conventional conductive gels are not necessary anymore.

Now, manufacturing of the sensor 10 will be briefly described.

The membrane 11 is a flexible film, and has the wire layer 16 on its surface, produced by using a typical flexible printed circuitry (FPC) method. The electrode 12 having the needle 12a is produced by using MEMS technologies such as a micro finger or an electro fine forging. The cohesive sheet 15 is made of a cohesive polymer, and has thru-holes 15a made in predetermined positions by using a punching machine. The thru-holes may be provided in predetermined positions of the magnets 14 as well as the electrodes 12. The cohesive polymer sheet 15 is protected by a release base paper, and one surface thereof is attached to the membrane 11. The electrode 12 is soldered into circuit sections of the membrane 11. For bonding the electrodes 12, a bonding area is provided on predetermined positions on the membrane 11.

In the above embodiment, detailed descriptions have not been provided for the transmitter 20 and the terminal 21 connected thereto. However, anyone skilled in the art will readily achieve them according to the conventional art.

The sensor 10 according to the present invention has an easily removable structure from the transmitter 20, does not use wires, and thus has a low noise level. In addition, due to a stable electrical connection structure, no complicated voltages or impedances are caused by an unstable contact between the electrodes 12 and the human skin. Particularly, since the sensor 10 has a structure in which a contact between the electrode 12 and the human skin can be achieved without using gel, and the needles 12a pass through the stratum corneum, it is possible to obtain accurate biomedical signals.

The present invention is suitable for a signal detection apparatus for living bodies, and, particularly, for a remote biomedical examination.

While the present invention has been particularly shown and described with reference to exemplary embodiments thereof, it will be understood by those skilled in the art that various changes in form and details may be made therein without departing from the scope of the invention as defined by the appended claims. The exemplary embodiments should be considered in descriptive sense only and not for purposes of limitation. Therefore, the scope of the invention is defined not by the detailed description of the invention but by the appended claims, and all differences within the scope will be construed as being included in the present invention.

## Claims

1. A body surface bio-potential sensor (10) comprising:
a flexible membrane (11) having a wire layer (16);
a plurality of electrodes (12) attached on one surface of the membrane at predetermined intervals, the electrodes having a plurality of needles (12a) of a predetermined height on a surface thereof;
a cohesive layer (15) covering one surface of the membrane except predetermined regions of the electrodes (12); and
a terminal (13) electrically connected to the electrodes for outputting electrical signals;
**characterised in that** the terminal is provided on the other surface of the membrane to the electrodes (12) for electrically coupling the electrodes (12) to a transmitter (20) for processing the signals;
**characterised in that** the membrane is provided with a permanent magnet (14) for combining the sensor (10) with a permanent magnet (22) in the transmitter (20).

2. The sensor according to claim 1, wherein the needles (129) have a height of 5µm or lower.

3. An apparatus for detecting biomechanical signals, the apparatus comprising:
the body surface bio-potential sensor (10) of any preceding claim; and
a transmitter (20) removably attached to the sensor to transmit electricalsignals from a multiple electrode patch as radio signals, wherein the transmitter (20) has a permanent magnet (22) for connecting with the permanent magnet (14) of the sensor providing a removable attaching structure.

4. The apparatus according to claim 3, wherein the sensor and the transmitter have terminals of complementary structures for delivering biomedical signals from the electrodes.

## Patentansprüche

1. Körperoberflächen-Biopotentialsensor (10), der Folgendes umfasst:
eine biegsame Membran (11) mit einer Drahtschicht (16);
mehrere Elektroden (12), die in vorherbestimmten Abständen auf einer Oberfläche der Membran befestigt sind, wobei die Elektroden mehrere Nadeln (12a) einer vorherbestimmten Höhe auf einer Oberfläche derselben aufweisen;
eine geschlossene Schicht (15), die eine Oberfläche der Membran außer vorherbestimmten Regionen der Elektroden (12) bedeckt; und
einen Anschluss (13), der elektrisch mit den Elektroden verbunden ist, um elektrische Signale auszugeben;
**dadurch gekennzeichnet, dass** der Anschluss auf der anderen Oberfläche der Membran vorgesehen ist als die Elektroden (12), um die Elektroden (12) elektrisch an einen Sender (20) zu koppeln, um die Signale zu verarbeiten;
**dadurch gekennzeichnet, dass** die Membran mit einem Permanentmagnet (14) ausgestattet ist, um den Sensor (10) mit einem Permanentmagnet (22) in dem Sender (20) zu kombinieren.

2. Sensor nach Anspruch 1, wobei die Nadeln (129) eine Höhe von 5 µm oder weniger aufweisen.

3. Vorrichtung zum Erkennen von biomechanischen Signalen, wobei die Vorrichtung Folgendes umfasst:
den Körperoberflächen-Biopotentialsensor (10) nach einem der vorangehenden Ansprüche; und
einen Sender (20), der abnehmbar an dem Sensor befestigt ist, um elektrische Signale von einem Pflaster mit mehreren Elektroden als Funksignale zu übertragen, wobei der Sender (20) einen Permanentmagnet (22) zum Verbinden mit dem Permanentmagnet (14) des Sensors aufweist, so dass eine abnehmbare Befestigungsstruktur vorgesehen ist.

4. Vorrichtung nach Anspruch 3, wobei der Sensor und der Sender Anschlüsse mit komplementären Strukturen aufweisen, um biomedizinische Signale von den Elektroden zu liefern.

## Revendications

1. Capteur (10) de biopotentiel à application sur une surface corporelle comprenant :
une membrane souple (11) ayant une couche de fils métalliques (16) ;
une pluralité d'électrodes (12) attachées sur une surface de la membrane à des intervalles prédéterminés, les électrodes ayant une pluralité d'aiguilles (12a) d'une hauteur prédéterminée sur l'une de leurs surfaces ;
une couche cohésive (15) recouvrant une surface de la membrane à l'exception de régions prédéterminées des électrodes (12) ; et
une borne (13) connectée électriquement aux électrodes pour produire en sortie des signaux électriques ;
**caractérisé en ce que** la borne est fournie sur l'autre surface de la membrane par rapport aux électrodes (12) pour coupler électriquement les électrodes (12) à un émetteur (20) en vue du traitement des signaux ;
**caractérisé en ce que** la membrane est dotée d'un aimant permanent (14) pour combiner le capteur (10) à un aimant permanent (22) dans l'émetteur (20).

2. Capteur selon la revendication 1, dans lequel les aiguilles (129) ont une hauteur de 5 µm ou moins.

3. Appareil de détection de signaux biomécaniques, l'appareil comprenant :
le capteur (10) de biopotentiel à application sur une surface corporelle selon l'une quelconque des revendications précédentes ; et
un émetteur (20) fixé de manière détachable au capteur afin d'émettre depuis un tampon d'électrodes multiples des signaux électriques sous forme de signaux radio, l'émetteur (20) ayant un aimant permanent (22) destiné à être connecté à l'aimant permanent (14) du capteur produisant une structure de fixation détachable.

4. Appareil selon la revendication 3, dans lequel le capteur et l'émetteur ont des bornes de structures complémentaires pour délivrer des signaux biomédicaux depuis les électrodes.
